# EUROPÄISCHE PATENTSCHRIFT

(11) **EP 2 785 319 B1**
(45) Veröffentlichungstag und Bekanntmachung des Hinweises auf die Patenterteilung: **17.05.2017**
(21) Anmeldenummer: 12775262.4
(22) Anmeldetag: 23.10.2012
(51) Int. Cl.: A61K 8/898, A61Q 5/00, A61Q 5/08, A61Q 5/10, C08G 77/26

(54) **VORBEHANDLUNGSMITTEL FÜR KERATINISCHE FASERN MIT 4-MORPHOLINOMETHYL-SUBSTITUIERTEN SILIKON(EN)**
PRETREATMENT AGENTS FOR KERATIN FIBERS COMPRISING 4-MORPHOLINO-METHYL-SUBSTITUTED SILICONE(S)
AGENTS DE PRÉTRAITEMENT POUR LES FIBRES KÉRATINIQUES COMPRENANT UNE OU PLUSIEURS SILICONES À SUBSTITUTION 4-MORPHOLINOMÉTHYLE

(30) Priorität: 29.11.2011 DE 102011087344
(43) Veröffentlichungstag der Anmeldung: 08.10.2014
(73) Patentinhaber: Henkel AG & Co. KGaA, 40589 Düsseldorf (DE)
(72) Erfinder: SCHULZE ZUR WIESCHE, Erik, 20144 Hamburg (DE); NOLL, Monika, 22851 Norderstedt (DE)
(86) Internationale Anmeldenummer: PCT/EP2012/070919
(87) Internationale Veröffentlichungsnummer: WO 2013/079258

(56) Entgegenhaltungen:
- EP-A2- 1 312 345
- "Kosmetische Zusammensetzungen", IP.COM JOURNAL, IP.COM INC., WEST HENRIETTA, NY, US, 4. August 2011 (2011-08-04), XP013146799, ISSN: 1533-0001

## Beschreibung

Die vorliegende Erfindung betrifft kosmetische Zusammensetzungen, die keratinische Fasern vor oxidativen Einflüssen schützen.

Beim Färben und beim Blondieren von Haaren tritt das Problem auf, daß es aufgrund der aggressiven Agentien zu Irritationen auf der Kopfhaut und Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind sogenannte Vorbehandlungsmittel auf dem Markt, die das Haar vor dem aggressiven Einfluß schützen sollen. Diese beschweren das Haar aber oft oder beeinträchtigen den Erfolg der im Anschluß stattfindenden Aufhellung bzw. Färbung des Haares.

Der vorliegenden Erfindung lag die Aufgabe zugrunde, Mittel bereitzustellen, die die genannten Nachteile überwinden, ohne den Erfolg einer nachfolgenden oxidativen Behandlung zu konterkarieren. Dabei sollten insbesondere Mittel bereitgestellt werden, die das Haar nicht beschweren und auch bei einer nicht unmittelbar vor der oxidativen Behandlung stattfindenden Vorbehandlung die gewünschte Wirkung erzielen, wodurch die Zeitspanne zwischen Vorbehandlung und Färben bzw. Blondieren verlängert werden kann.

Der Einsatz von aminierten Silikonen in der Haarpflege ist Stand der Technik. Diese werden in Shampoos und insbesondere in Conditionern breit eingesetzt, um dort Pflegeffekte zu entfalten. So offenbart die EP 1 771 144 B1 haarkonditionierende Mittel mit aminofunktionellen Siliconen. Die dort beschriebenen Mittel sind Nachbehandlungsmittel.

Auch die europäischen Patente EP 1 312 334 B1 (Aminosilicon und Verdicker) sowie EP 1 312 335 B1 (Aminosilicon und Conditioner) offenbaren Haarnachbehandlungsmittel. Im erstgenannten Dokument werden auch extrem wasserreiche Rezepturen offenbart.

Die europäische Patentanmeldung EP 1 312 345 A2 offenbart Vorbehandlungsmittel für oxidative Haarbehandlungsprozesses, welche Amodimethicone enthalten.

Im IP.COM Journal August 2011 werden unter XP013146799 Beispielformulierungen für Haarpflegeprodukte offenbart, welche hohe Mengen an Wasser und morpholinomethyl-substituierte Amodimethicone enthalten.

Es wurde nun gefunden, daß eine Vorbehandlung der keratinischen Fasern mit speziellen 4-Morpholinomethyl-substituierten Silikonen innerhalb eines bestimmten Zeitraums vor einer oxidativen Behandlung zu deutlich verbessertem Haarschutz führt, ohne daß die Ergebnisse der oxidativen Behandlung beeinträchtigt werden.

Gegenstand der vorliegenden Erfindung ist in einer ersten Ausführungsform ein Verfahren zur oxidativen Haarbehandlung, dadurch gekennzeichnet, daß
a) ein Vorbehandlungsmittel auf die keratinischen Fasern aufgetragen wird, das bezogen auf sein Gewicht
   a) 0,00001 bis 20 Gew.-% mindestens eines 4-morpholinomethyl-substituierten Silikons enthält, das jeweils mindestens eine der Struktureinheiten der Formeln (I), (II) und (III) aufweist 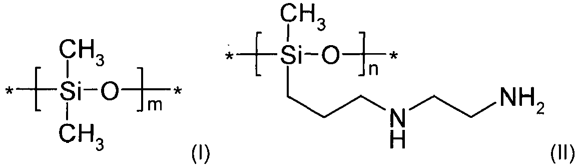 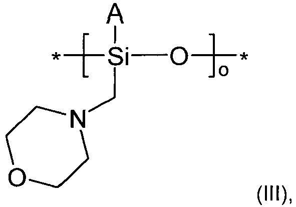 worin
      * für eine Bindung zu einer der Struktureinheiten (I), (II) oder (III) oder für eine Endgruppe B (Si-gebunden) oder D (O-gebunden) steht,
      B für eine Gruppe -OH, -O-Si(CH₃)₃, -O-Si(CH₃)₂OH, -O-Si(CH₃)₂OCH₃ steht,
      D für eine Gruppe -H; -Si(CH₃)₃, -Si(CH₃)₂OH, -Si(CH₃)₂OH₃ steht,
      A für eine über ein -O- gebundene Struktureinheit (I) oder einen über ein -O-gebundenen oligomeren oder polymeren Rest enthaltend Struktureinheiten der Formel (I) oder für -OH steht,
      n, m und o für ganze Zahlen zwischen 1 und 1000 stehen.
   b) mindestens 50 Gew.-% Wasser enthält
b) die keratinischen Fasern innerhalb eines Zeitraumes von einer Sekunde bis 24 Stunden nach Schritt a) einer oxidativen Haarbehandlung unterworfen werden.

Die im erfindungsgemäßen Verfahren eingesetzten Vorbehandlungsmittel sind wasserbasiert und enthalten mindestens 80 Gew.-% Wasser. Bevorzugteim erfindungsgemäßen Verfahren eingesetzte Mittel enthalten noch höhere Wassermengen, bis hin zu 99 Gew.-%. Besonders bevorzugte im erfindungsgemäßen Verfahren eingesetzte Mittel sind dadurch gekennzeichnet, daß sie, bezogen auf das Gewicht des Mittels, 70 bis 99,9 Gew.%, vorzugsweise 80 bis 99,5 Gew.%, besonders bevorzugt 85 bis 99,25 Gew.% und insbesondere 90 bis 98 Gew.% Wasser enthalten.

Als Aktivstoff enthalten die im erfindungsgemäßen Verfahren eingesetzten Mittel 0,00001 bis 10 Gew.-% mindestens eines 4-morpholinomethyl-substituierten Silikons, das jeweils mindestens eine der Struktureinheiten der Formeln (I), (II) und (III) aufweist.

Die Struktureinheiten der Formeln (I), (II) und (III) können dabei statistisch verteilt im Molekül vorliegen, die erfindungsgemäß eingesetzten Silikone können aber auch Blockcopolymere aus Blöcken der einzelnen Struktureinheiten sein, wobei die Blöcke wiederum statistisch verteilt vorliegen können.

Der * an den freien Valenzen der Struktureinheiten (I), (II) oder (III) steht dabei für eine Bindung zu einer der Struktureinheiten (I), (II oder (III) oder für eine Endgruppe B (Si-gebunden) oder D (O-gebunden).

Die erfindungsgemäß eingesetzten Silikone können beidseitig trimethylsilyl-terminiert sein (D =-Si(CH₃)₃, B = -O-Si(CH₃)₃), sie können aber auch ein- oder zweiseitig dimethylsilylhydroxy- oder dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B =-O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, insbesondere zu einer gravierenden Verringerung des Kontaktwinkels. In der Struktureinheit (III) kann der Rest A
- für eine über ein -O- gebundene Struktureinheit (I) oder
- einen über ein -O- gebundenen oligomeren oder polymeren Rest enthaltend Struktureinheiten der Formel (I)
- oder für-OH stehen.

Im erstgenannten Fall wird die Struktureinheit (III) zu der Struktureinheit (IIIa): 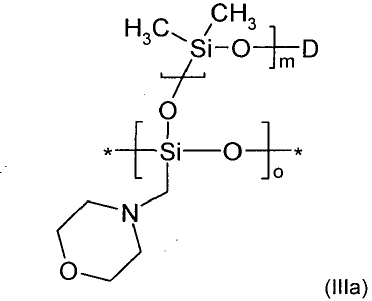 mit m = n = o = 1 und A bzw. D wie vorstehend definiert.

Im zweiten Fall können in der vorstehend genannten Formel (IIIa) die Indices m, n und o für ganze Zahlen zwischen 2 und 1000 stehen.

Im dritten Fall steht A für-OH (dargestellt in der Struktureinheit (IIIf)

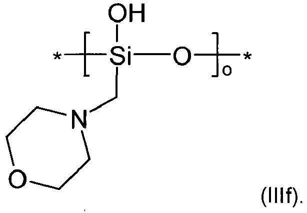

Wie bereits erwähnt, können die Struktureinheiten der Formeln (I), (II) und (III) vorzugsweise statistisch verteilt vorliegen. Erfindungsgemäß bevorzugte Vorbehandlungsmittel enthalten mindestens ein 4-morpholinomethyl-substituiertes Silikon der Formel (V) 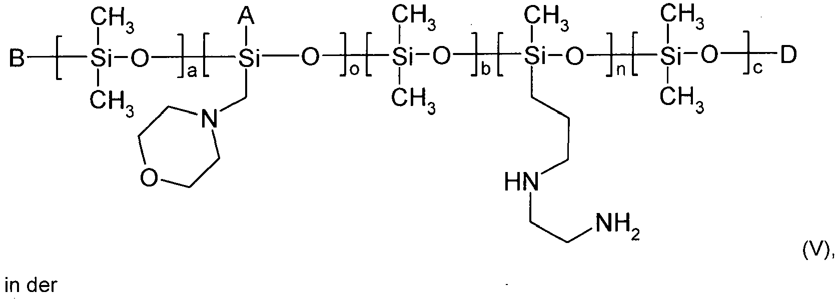
A für eine über ein -O- gebundene Struktureinheit (I) oder einen über ein -O-gebundenen oligomeren oder polymeren Rest enthaltend Struktureinheiten der Formel (I) oder für-OH steht,
B für eine Gruppe -OH, -O-Si(CH₃)₃, -O-Si(CH₃)₂OH, -O-Si(CH₃)₂OCH₃ steht,
D für eine Gruppe -H; -Si(CH₃)₃, -Si(CH₃)₂OH, -Si(CH₃)₂OCH₃ steht,
a, b und c für ganze Zahlen zwischen 0 und 1000 stehen, mit der Maßgabe a + b + c > 0
n und o für ganze Zahlen zwischen 1 und 1000 stehen.

Strukturformel (V) soll verdeutlichen, daß die Siloxangruppen n und o nicht zwingend direkt an eine Endgruppierung B bzw. D gebunden sein müssen. Vielmehr gilt in bevorzugten Formeln (V) a > 0 oder b > 0 und in besonders bevorzugten Formeln (V) a > 0 und b > 0, d.h. die terminale Gruppierung B bzw. D ist vorzugsweise an eine Dimethylsiloxy-Gruppierung gebunden. Auch in Formel (V) sind die Siloxaneinheiten a, b, c, n und o vorzugsweise statistisch verteilt.

Auch die durch Formel (V) dargestellten erfindungsgemäß eingesetzten Silikone können beidseitig trimethylsilyl-terminiert sein (D = -Si(CH₃)₃, B = -O-Si(CH₃)₃), sie können aber auch ein- oder zweiseitig dimethylsilylhydroxy- oder dimethylsilylmethoxy-terminiert sein. Im Rahmen der vorliegenden Erfindung besonders bevorzugt eingesetzte Silikone weisen mindestens eine endständige Dimethylsilylhydroxy-Gruppe auf, d.h. sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, insbesondere zu einem gravierend verbesserten Schutz bei oxidativer Behandlung.

Auch in Formel (V) kann der Rest A
- für eine über ein -O- gebundene Struktureinheit (I) oder
- einen über ein -O- gebundenen oligomeren oder polymeren Rest enthaltend Struktureinheiten der Formel (I)
- oderfür-OH stehen.

Analog den Ausführungen zur Struktureinheit (III) wird damit Formel (V) präzisiert zu einer der Formeln (Va) oder (Vf), wie im Prioritätsdokument auf den Seiten 6 bis 8 offenbart.

Unabhängig davon, welches spezielle 4-Morpholinomethyl-substituierte Silikon in den im erfindungsgemäßen Verfahren eingesetzten Vorbehandlungsmitteln eingesetzt wird, sind im erfindungsgemäßen Verfahren eingesetzte Mittel bevorzugt, die ein 4-Morpholinomethyl-substituiertes Silikon enthalten, in dem mehr als 50 Mol-% der Struktureinheiten Dimethylsiloxy-Einheiten sind, d.h. in denen die Struktureinheit (I) mindestens die Hälfte aller Struktureinheiten des eingesetzten Silikons ausmacht.

In anderen Worten sind Silikone bevorzugt, bei denen m > (n + o) bzw. (a+b+c) > (n + o) gilt.

Noch weiter bevorzugte Vorbehandlungsmittel enthalten ein 4-Morpholinomethyl-substituiertes Silikon, in dem mehr als 90 Mol-% der Struktureinheiten Dimethylsiloxy-Einheiten sind, d.h. in denen die Struktureinheit (I) mindestens Neun zehntel aller Struktureinheiten des eingesetzten Silikons ausmacht.

In anderen Worten sind Silikone bevorzugt, bei denen m > 10(n + o) bzw. (a+b+c) > 10(n + o) gilt.

Noch weiter bevorzugte Vorbehandlungsmittel enthalten ein 4-Morpholinomethyl-substituiertes Silikon, in dem mehr als 98 Mol-% der Struktureinheiten Dimethylsiloxy-Einheiten sind, d.h. in denen die Struktureinheit (I) mindestens Achtundneunzig Hundertstel aller Struktureinheiten des eingesetzten Silikons ausmacht.

In anderen Worten sind Silikone bevorzugt, bei denen m > 50(n + o) bzw. (a+b+c) > 50(n + o) gilt.

Noch weiter bevorzugte Vorbehandlungsmittel enthalten ein 4-Morpholinomethyl-substituiertes Silikon, in dem mehr als 98,5 Mol-% der Struktureinheiten Dimethylsiloxy-Einheiten sind, d.h. in denen die Struktureinheit (I) mindestens Neunhundertfünfundachtzig Tausendst aller Struktureinheiten des eingesetzten Silikons ausmacht.

In anderen Worten sind Silikone bevorzugt, bei denen m > 75(n + o) bzw. (a+b+c) > 75(n + o) gilt.

Noch weiter bevorzugte Vorbehandlungsmittel enthalten ein 4-Morpholinomethyl-substituiertes Silikon, in dem mehr als 99 Mol-% der Struktureinheiten Dimethylsiloxy-Einheiten sind, d.h. in denen die Struktureinheit (I) mindestens Neun zehntel aller Struktureinheiten des eingesetzten Silikons ausmacht. In anderen Worten sind Silikone bevorzugt, bei denen m > 100(n + o) bzw. (a+b+c) > 100(n + o) gilt.

Zusammenfassend sind bevorzugte im erfindungsgemäßen Verfahren eingesetzte Vorbehandlungsmittel dadurch gekennzeichnet, daß sie mindestens ein 4-morpholinomethyl-substituiertes Silikon enthalten, bei dem
- m > (n + o) bzw. (a+b+c) > (n + o), vorzugsweise
- m > 10(n + o) bzw. (a+b+c) > 10(n + o), besonders bevorzugt
- m > 50(n + o) bzw. (a+b+c) > 50(n + o), weiter bevorzugt
- m > 75(n + o) bzw. (a+b+c) > 75(n + o) und insbesondere
- m > 100(n + o) bzw. (a+b+c) > 100(n + o) gilt.

Das bzw. die 4-Morpholinomethyl-substituierten Silikon(e) können je nach Anwendungszweck der im erfindungsgemäßen Verfahren eingesetzten Mittel in variierenden Mengen eingesetzt werden.

Bevorzugte im erfindungsgemäßen Verfahren eingesetzte Vorbehandlungsmittel sind dadurch gekennzeichnet, daß sie - bezogen auf ihr Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% 4-morpholinomethyl-substituierte(s) Silikon(e) enthalten.

Es hat sich gezeigt, daß die Wirkung der im erfindungsgemäßen Verfahren eingesetzten Silikone noch gesteigert werden kann, wenn bestimmte nichtionische Komponenten ebenfalls in den im erfindungsgemäßen Verfahren eingesetzten Mitteln eingesetzt werden. Zudem haben diese nichtionischen Komponenten positive Effekte auf die Lagerstabilität der im erfindungsgemäßen Verfahren eingesetzten Mittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die im erfindungsgemäßen Verfahren eingesetzten Mittel inkorporiert werden. Erfindungsgemäß besonders bevorzugte Vorbehandlungsmittel enthalten - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-w-hydroxypolyglycolether (INCI-Bezeichnung: Trideceth-10) oder deren Mischungen.

Weitere Tenside und Emulgatoren sind in den im erfindungsgemäßen Verfahren eingesetzten Mitteln vorzugsweise nicht oder nur in geringen Mengen enthalten. Erfindungsgemäß bevorzugte Mittel werden tensidarm formuliert, wobei bevorzugte Mittel ≤ 5 Gew.-% Tensid(e), vorzugsweise ≤ 2,5 Gew.-% Tensid(e), weiter bevorzugt ≤ 1 Gew.-% Tensid(e) und insbesondere ≤ 0,5 Gew.-% Tensid(e) enthalten. In diese Tensidmenge sind die Verbindungen der Formel (II) mit einberechnet.

Vorzugsweise werden die im erfindungsgemäßen Verfahren eingesetzten Mittel niedrigviskos formuliert. Es hat sich darüber hinaus gezeigt, daß verdickende Polymere die erfindungsgemäße Wirkung abschwächen können, so daß bevorzugte im erfindungsgemäßen Verfahren eingesetzte Mittel dadurch gekennzeichnet sind, daß sie ≤ 2,5 Gew.-%, vorzugsweise ≤ 1 Gew.-%, weiter bevorzugt ≤ 0,5w.-% und insbesondere ≤ 0,01 Gew.-% verdickende(s) Polymer(e) enthalten.

Mit besonderem Vorzug enthalten die im erfindungsgemäßen Verfahren eingesetzten Mittel unabhängig von der speziellen Wirkung des/der Polymer(e) sie ≤ 2,5 Gew.-%, vorzugsweise ≤ 1 Gew.-%, weiter bevorzugt ≤ 0,5w.-% und insbesondere ≤ 0,01 Gew.-% synthetische(s) Polymer(e)

Die im erfindungsgemäßen Verfahren eingesetzten Vorbehandlungsmittel können weitere Inhaltsstoffe enthalten. Bevorzugt ist hierbei der Einsatz von mehrwertigen Alkoholen, die feuchtigkeitsspendende Eigenschaften aufweisen. Hier sind im erfindungsgemäßen Verfahren eingesetzte Mittel bevorzugt, die - bezogen auf ihr Gewicht - 0,05 bis 15 Gew.-%, vorzugsweise 0,1 bis 10 Gew.-%, besonders bevorzugt 0,15 bis 5 Gew.-% und insbesondere 0,15 bis 1 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Glycerin bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Glycerin, so daß Mittel, die außer Glycerin keine anderen mehrwertigen Alkohole enthalten, besonders bevorzugt sind.

Im Hinblick auf die Vorbehandlung vor einer oxidativen Behandlung ist der Einsatz bestimmter Pflegestoffe in den im erfindungsgemäßen Verfahren eingesetzten Mitteln bevorzugt.

Im erfindungsgemäßen Verfahren bevorzugt eingesetzte Vorbehandlungsmittel sind dadurch gekennzeichnet, daß sie zusätzlich Pflegestoff(e) - bezogen auf ihr Gewicht- in Mengen von 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% enthalten, wobei bevorzugte Pflegstoff(e) ausgewählt sind aus der Gruppe
i. L-Carnitin und/oder seiner Salze;
ii. Taurin und/oder seiner Salze;
iii. Niacinamid;
iv. Ubichinon
v. Ectoin;

L-Carnitin (IUPAC-Name(R)-(3-Carboxy-2-hydroxypropyl)- N,N,N-trimethylammoniumhydroxid), ist eine natürlich vorkommende, vitaminähnliche Substanz. Als Betain kann L-Carnitin Additionsverbindungen und Doppelsalze bilden. Erfindungsgemäß bevorzugte L-Carnitinderivate sind insbesondere ausgewählt aus Acetyl-L-Carnitin, L-Carnitin-Fumarat, L-Carnitin-Citrat, Lauroyl-L-Carnitin und besonderes bevorzugt L-Carnitin-Tartrat. Die genannten L-Carnitin-Verbindungen sind beispielsweise von der Firma Lonza GmbH (Wuppertal, Deutschland) erhältlich.

Im erfindungsgemäßen Verfahren bevorzugt eingesetzte Haarbehandlungsmittel sind dadurch gekennzeichnet, daß sie - bezogen auf ihr Gewicht-0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.

Ein weiterer, bevorzugter einsetzbarer Pflegestoff ist das Taurin. Im erfindungsgemäßen Verfahren bevorzugt eingesetzte Vorbehandlungsmittel enthalten - bezogen auf ihr Gewicht- 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).

Eine weitere bevorzugte Gruppe von Pflegestoffen in den im erfindungsgemäßen Verfahren eingesetzten Mitteln sind Vitamine, Provitamine oder Vitaminvorstufen. Diese werden nachfolgend beschrieben:

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt (siehe weiter unten). Erfindungsgemäß einsetzbare Derivate des Panthenols sind insbesondere die Ester und Ether des Panthenols sowie kationisch derivatisierte Panthenole. Die genannten Verbindungen des Vitamin B₅-Typs sind in den erfindungsgemäßen Mitteln bevorzugt in Mengen von 0,05 -10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den im erfindungsgemäßen Verfahren eingesetzten Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den im erfindungsgemäßen Verfahren eingesetzten Mitteln bevorzugt in Mengen von 0,0001 bis 1,0 Gew.-%, insbesondere in Mengen von 0,001 bis 0,01 Gew.-% enthalten.

Zusammenfassend sind im erfindungsgemäßen Verfahren eingesetzte Haarbehandlungsmittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es hat sich gezeigt, daß bestimmte Chinone eine besondere Eignung als Pflegestoff besitzen. Als weiteren Pflegestoff können die im erfindungsgemäßen Verfahren eingesetzten Mittel daher 0,0001 bis 5 Gew.-% mindestens eines Biochinons der Formel (Ubi) 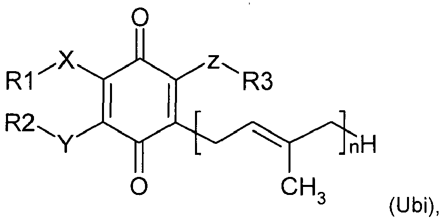 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- R⁴: steht für -CH₃, -CH₂CH₃, -(CH₂)₂CH₂, -CH(CH₃)₂, - (CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte im erfindungsgemäßen Verfahren eingesetzte Vorbehandlungsmittel sind dadurch gekennzeichnet, daß sie als Pflegestoff - bezogen auf ihr Gewicht - 0,0001 bis 1 Gew.-%, bevorzugt 0,001 bis 0,5

Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 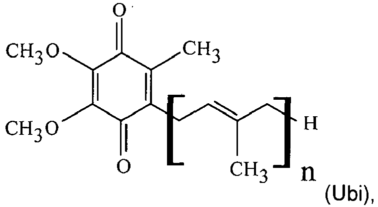 in der n für die Werte = 6, 7, 8, 9 oder 10, besonders bevorzugt für 10 (Coenzym Q10) steht.

Alternativ zu den besonders bevorzugten Ubichinonen oder zusätzlich zu ihnen können die im erfindungsgemäßen Verfahren eingesetzten Mittel auch Plastochinone enthalten. Hier sind bevorzugte im erfindungsgemäßen Verfahren eingesetzte Mittel dadurch gekennzeichnet, daß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 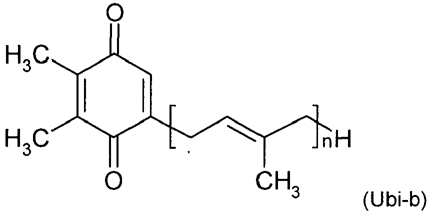 in der n für Werte von 1 bis 20, vorzugsweise von 2 bis 15 und insbesondere für 5, 6, 7, 8, 9, 10 steht, wobei besonders bevorzugt Mittel Plastochinon PQ-9 der Formel 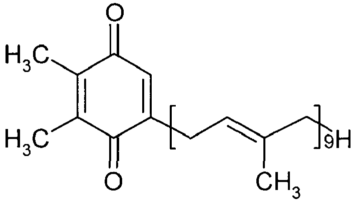 enthalten.

Als weiteren Pflegestoff können die im erfindungsgemäßen Verfahren eingesetzten Mittel Ectoin ((4S)-2-Methyl-1,4,5,6-Tetrahydropyrimidin-4-Carbonsäure) enthalten. Im erfindungsgemäßen Verfahren bevorzugt eingesetzte Vorbehandlungsmittel sind dadurch gekennzeichnet, daß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.

Zur Verbesserung der Elastizität und Festigung der inneren Struktur der mit im erfindungsgemäßen Verfahren eingesetzten Mitteln vorbehandelter Haare können die im erfindungsgemäßen Verfahren eingesetzten Mittel Purin und/oder Purinderivate als Pflegestoff enthalten. Insbesondere die Kombination von Purin und/oder Purinderivaten mit Ubichinonen und/oder Plastochinonen als Pflegestoff führt dazu, daß die nachfolgende oxidative Behandlung schadlos überstanden wird.

Purin (7*H*-Imidazo[4,5-d]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden.

Purine und Purinderivate werden oft aus Naturstoffen isoliert, sind aber auch auf vielen Wegen synthetisch zugänglich.

Bevorzugte im erfindungsgemäßen Verfahren eingesetzte Mittel enthalten Purin und/oder Purinderivate in engeren Mengenbereichen. Hier sind im erfindungsgemäßen Verfahren bevorzugt eingesetzte Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.

Unter Purin, den Purinen und den Purinderivaten sind erfindungsgemäß einige Vertreter besonders bevorzugt. Im erfindungsgemäßen Verfahren bevorzugt eingesetzte Vorbehandlungsmittel sind dadurch gekennzeichnet, daß sie als Pflegestoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 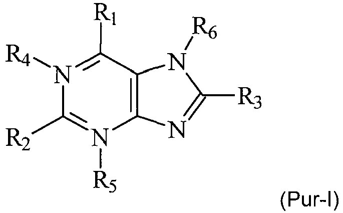 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Es ist weiterhin vorteilhaft, Purin bzw. Purinderivate und Biochinone in einem bestimmten Verhältnis zueinander einzusetzen. Hier sind im erfindungsgemäßen Verfahren eingesetzte Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.

Wie bereits erwähnt, ist Coffein ein besonders bevorzugtes Purinderivat, und das Coenzym Q10 ist ein besonders bevorzugtes Biochinon. Besonders bevorzugte im erfindungsgemäßen Verfahren eingesetzte Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.

Als Pflegestoff können die im erfindungsgemäßen Verfahren eingesetzten Mittel auch Flavonoide enthalten. Die Flavonoide sind eine Gruppe von wasserlöslichen Pflanzenfarbstoffen und spielen eine wichtige Rolle im Stoffwechsel vieler Pflanzen. Sie gehören zusammen mit den Phenolsäuren zu den Polyphenolen. Es sind weit über 6500 unterschiedliche Flavonoide bekannt, die sich in Flavonole, Flavone, Flavanone, Isoflavonoide und Anthocyane einteilen lassen.

Erfindungsgemäß können Flavonoide aus allen sechs Gruppen eingesetzt werden, wobei bestimmte Vertreter aus den einzelnen Gruppen als Pflegestoff wegen ihrer besonders intensiven Wirkung bevorzugt sind. Bevorzugte Flavonole sind Quercetin, Rutin, Kaempferol, Myricetin, Isorhamnetin, bevorzugte Flavanole sind Catechin, Gallocatechin, Epicatechin, Epigallocatechingallat , Theaflavin, Thearubigin, bevorzugte Flavone sind Luteolin, Apigenin, Morin, bevorzugte Flavanone sind Hesperetin, Naringenin, Eriodictyol, bevorzugte Isoflavonoide sind Genistein, Daidzein, und bevorzugte Anthocyanidine (Anthocyane) sind Cyanidin, Delphinidin, Malvidin, Pelargonidin, Peonidin, Petunidin.

Im erfindungsgemäßen Verfahren besonders bevorzugt eingesetzte Vorbehandlungsmittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-O-rutinosid (Rutin), enthalten.

Bevorzugt ist auch der Einsatz von Bisabolol und/oder Bisabololoxiden als Pflegestoff in den im erfindungsgemäßen Verfahren eingesetzten Mitteln. Hier sind im erfindungsgemäßen Verfahren eingesetzte Vorbehandlungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.

Auch Creatin eignet sich erfindungsgemäß als Pflegestoff. Creatin (3-Methylguanidinoessigsäure) ist eine organische Säure, die in Wirbeltieren u. a. zur Versorgung der Muskeln mit Energie beiträgt. Kreatin wird in der Niere, der Leber und in der Bauchspeicheldrüse synthetisiert. Sie leitet sich formal von den Aminosäuren Glycin und Arginin ab und ist zu 95 % im Skelettmuskel vorhanden. Besonders bevorzugte erfindungsgemäße Vorbehandlungsmittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% *N*-Methyl-guanidino-essigsäure (Creatin).

Zusätzlich zu den Pflegestoffen können die im erfindungsgemäßen Verfahren eingesetzten Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren.

Als weiteren Inhaltsstoff können die im erfindungsgemäßen Verfahren eingesetzten Mittel mit besonderem Vorzug eine oder mehrere Aminosäuren enthalten. Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte im erfindungsgemäßen Verfahren eingesetzte Mittel enthalten eine oder mehrere Aminosäuren in engeren Mengenbereichen. Hier sind im erfindungsgemäßen Verfahren bevorzugt eingesetzte Haarbehandlungsmittel dadurch gekennzeichnet, daß sie als Pflegestoff - bezogen auf ihr Gewicht - 0,01 bis 5 Gew.-%, vorzugsweise 0,02 bis 2,5 Gew.-%, besonders bevorzugt 0,05 bis 1,5 Gew.-%, weiter bevorzugt 0,075 bis 1 Gew.-% und insbesondere 0,1 bis 0,25 Gew.-% Aminosäure(n), vorzugsweise aus der Gruppe Glycin und/oder Alanain und/oder Valin und/oder Lysin und/oder Leucin und/oder Threonin enthalten.

Als weiteren Bestandteil können die im erfindungsgemäßen Verfahren eingesetzten Mittel mindestens ein Kohlenhydrat aus der Gruppe der Monosaccharide, Disaccharide und/oder Oligosaccharide enthalten. Hier sind im erfindungsgemäßen Verfahren bevorzugte eingesetzte Haarbehandlungsmittel dadurch gekennzeichnet, daß sie als Pflegestoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus Monosachhariden, insbesondere D-Ribose und/oder D-Xylose und/oder L-Arabinose und/oder D-Glucose und/oder D-Mannose und/oder D-Galactose und/oder D-Fructose und/oder Sorbose und/oder L-Fucose und/oder L-Rhamnose sowie Disacchariden, insbesondere Saccharose und/oder Maltose und/oder Lactose und/oder Trehalose und/oder Cellobiose und/oder Gentiobiose und/oder Isomaltose.

Besonders bevorzugte im erfindungsgemäßen Verfahren eingesetzte Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.

Wie bereits erwähnt, enthalten bevorzugte im erfindungsgemäßen Verfahren eingesetzte Mittel (eine) Aminosäure(n).

Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte im erfindungsgemäßen Verfahren eingesetzte Mittel enthalten eine oder mehrere Aminosäuren in engeren Mengenbereichen. Hier sind im erfindungsgemäßen Verfahren bevorzugt eingesetzte Mittel dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.

Besonders bevorzugte im erfindungsgemäßen Verfahren eingesetzte Mittel enthalten bezogen auf ihr Gewicht
- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,
- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.

Das erfindungsgemäße Verfahren umfaßt das Aufbringen eines Vorbehandlungsmittels auf keratinische Fasern und eine sich daran anschließende oxidative Behandlung. Ein großer Vorteil der in Schritt a) eingesetzten Mittel ist es, daß sie nicht nur dann wirksam sind, wenn sie unmittelbar vor der oxidativen Behandlung angewendet werden, sondern bis zu 24 Stunden vorher angewendet werden können, ohne daß durch äußere Einflüsse eine Abschwächung der Wirkung zu befürchten wäre. Auf diese Weise ist es möglich, beispielsweise morgens nach der Haarwäsche Schritt a) des erfindungsgemäßen Verfahrens durchzuführen und die oxidative Behandlung erst abends.

Eine oxidative Haarbehandlung kann der Farb- und/oder Formveränderung der keratinischen Fasern dienen. Im Falle der Formveränderung (Dauerwelle, Glätten) wird das Haar vor der oxidativen Behandlung (Fixierung) mit einem weiteren Mittel behandelt, um die Faserstruktur formbar zu machen. In erfindungsgemäß bevorzugten Verfahren dient die oxidative Behandlung der Farbveränderung, stellt also eine Aufhellung ("Blondierung") oder ein Färben dar.

Erfindungsgemäß bevorzugte Verfahren sind dadurch gekennzeichnet, daß Schritt b) das Aufbringen eines Mittels zum Blondieren menschlicher Haare umfaßt, welches mindestens eine Peroxoverbindung enthält, die ausgewählt ist aus Wasserstoffperoxid und dessen Anlagerungsverbindungen an feste Träger, Ammonium- und Alkalimetall-persulfaten und -peroxidisulfaten.

In dem in Schritt b) des erfindungsgemäßen Verfahrens eingesetzten Blondiermittel ist Wasserstoffperoxid enthalten. Bevorzugt wird Wasserstoffperoxid selbst als wässrige Lösung verwendet. Das Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, eingesetzt werden.

Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet. Erfindungsgemäß bevorzugte Verfahren sind dadurch gekennzeichnet, daß die in Schritt b) eingesetzten Mittel - bezogen auf ihr Gewicht- 0,5 bis 12

Gew.-%, vorzugsweise 2 bis 10 Gew.-%, besonders bevorzugt 3 bis 6 Gew.-% Wasserstoffperoxid (berechnet als 100%iges H₂O₂) enthalten.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des die in Schritt b) eingesetzten Mittels zwischen 7 und 11, insbesondere zwischen 8 und 10,5 , liegt.

Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe Ammoniak, basischen Aminosäuren, Alkalihydroxiden, Alkanolaminen, Alkalimetallmetasilikaten, Alkaliphosphaten und Alkalihydrogenphosphaten ausgewählt werden. Als Alkalimetallionen dienen bevorzugt Lithium, Natrium, Kalium, insbesondere Natrium oder Kalium.

Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Für die starke Aufhellung sehr dunklen Haares ist der Einsatz von Wasserstoffperoxid oder dessen Anlagerungsprodukten an organische bzw. anorganische Verbindungen oftmals nicht ausreichend. In diesen Fällen wird in der Regel eine Kombination aus Wasserstoffperoxid und Persulfaten eingesetzt.

Die Persulfatsalze können in einer Menge von 0,1 bis 25 g, insbesondere in einer Menge von 1 bis 15 g, bezogen auf 100 g des anwendungsbereiten Mittels, enthalten sein.

Bevorzugte Persulfatsalze sind Ammoniumperoxidisulfat, Kaliumperoxidisulfat, Natriumperoxidisulfat, Ammoniumpersulfat, Kaliumpersulfat und Natriumpersulfat.

Alternativ kann das erfindungsgemäße Verfahren auch bei der Haarfärbung eingesetzt werden. In solchen bevorzugten Ausführungsformen ist das erfindungsgemäße Verfahren dadurch gekennzeichnet, daß Schritt b) das Aufbringen eines Haarfärbemittels umfaßt, welches mindestens ein Oxidationsfarbstoffvorprodukt und mindestens ein Oxidationsmittel enthält.

Als ersten zwingenden Inhaltsstoff enthalten die in Schritt b) dieses erfindungsgemäßen Verfahrens eingesetzten Mittel daher mindestens ein Oxidationsfarbstoffvorprodukt. Oxidationsfarbstoffvorprodukte können aufgrund ihres Reaktionsverhaltens in zwei Kategorien eingeteilt werden, sogenannte Entwicklerkomponenten und Kupplerkomponenten.

Entwicklerkomponenten können mit sich selbst den eigentlichen Farbstoff ausbilden. Sie können daher als alleinige, farbverändernde Verbindungen im erfindungsgemäßen Mittel enthalten sein. In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxy-ethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Ganz besonders bevorzugte Entwicklerkomponenten sind dabei p-Toluylendiamin, 2-(2-Hydroxy-ethyl)-p-phenylendiamin, 2-Methoxymethyl-p-phenylendiamin, N-(4-Amino-3-methylphenyl)-N-[3-(1 H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- 3-Aminophenol (m-Aminophenol) und/oder dessen Derivate,
- 3-Aminoanilin (m-Diaminobenzol) und/oder dessen Derivate,
- 2-Aminoanilin (1,2-Diaminobenzol; o-Diaminobenzol) und/oder dessen Derivate,
- 2-Aminophenol (o-Aminophenol) und/oder dessen Derivate,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,

Gemische aus zwei oder mehreren Verbindungen aus einer oder mehreren dieser Klassen sind im Rahmen dieser Ausführungsform ebenso erfindungsgemäß.

Erfindungsgemäß besonders bevorzugte Kupplerkomponenten werden ausgewählt unter 3-Amino-phenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Tri-hydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxy-indolin, 7-Hydroxyindolin oder Gemischen dieser Verbindungen oder den physiologisch verträglichen Salzen der vorgenannten Verbindungen.

Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Im Rahmen der vorliegenden Erfindung sind folgende Kombinationen aus Oxidationsfarbstoffvorprodukten vom Entwicklertyp und vom Kupplertyp besonders bevorzugt Mit den als Kombination genannten Oxidationsfarbstoffvorprodukten können jedoch auch noch weitere Farbstoffvorprodukte kombiniert werden:
p-Toluylendiamin / Resorcin;
p-Toluylendiamin / 2-Methylresorcin;
p-Toluylendiamin / 5-Amino-2-methylphenol;
p-Toluylendiamin / 3-Aminophenol;
p-Toluylendiamin / 2-(2,4-Diaminophenoxy)ethanol;
p-Toluylendiamin / 1,3-Bis(2,4-diaminophenoxy)propan;
p-Toluylendiamin / 1-Methoxy-2-amino-4-(2-hydroxyethylamino)benzol;
p-Toluylendiamin / 2-Amino-3-hydroxypyridin;
p-Toluylendiamin / 1-Naphthol;
2-(2-Hydroxyethyl)-p-phenylendiamin / Resorcin;
2-(2-Hydroxyethyl)-p-phenylendiamin / 2-Methylresorcin;
2-(2-Hydroxyethyl)-p-phenylendiamin / 5-Amino-2-methylphenol;
2-(2-Hydroxyethyl)-p-phenylendiamin / 3-Aminophenol;
2-(2-Hydroxyethyl)-p-phenylendiamin / 2-(2,4-Diaminophenoxy)ethanol;
2-(2-Hydroxyethyl)-p-phenylendiamin / 1,3-Bis(2,4-diaminophenoxy)propan;
2-(2-Hydroxyethyl)-p-phenylendiamin / 1-Methoxy-2-amino-4-(2-hydroxyethylamino)benzol;
2-(2-Hydroxyethyl)-p-phenylendiamin / 2-Amino-3-hydroxypyridin;
2-(2-Hydroxyethyl)-p-phenylendiamin / 1-Naphthol;
2-Methoxymethyl-p-phenylendiamin / Resorcin;
2-Methoxymethyl-p-phenylendiamin / 2-Methylresorcin;
2-Methoxymethyl-p-phenylendiamin / 5-Amino-2-methylphenol;
2-Methoxymethyl-p-phenylendiamin / 3-Aminophenol;
2-Methoxymethyl-p-phenylendiamin / 2-(2,4-Diaminophenoxy)ethanol;
2-Methoxymethyl-p-phenylendiamin / 1,3-Bis(2,4-diaminophenoxy)propan;
2-Methoxymethyl-p-phenylendiamin / 1-Methoxy-2-amino-4-(2-hydroxyethylamino)benzol;
2-Methoxymethyl-p-phenylendiamin / 2-Amino-3-hydroxypyridin;
2-Methoxymethyl-p-phenylendiamin / 1-Naphthol;
N-(4-Amino-3-methylphenyl)-N-[3-(1H-imidazol-1-yl)propyl]amin / Resorcin;
N-(4-Amino-3-methylphenyl)-N-[3-(1H-imidazol-1-yl)propyl]amin / 2-Methylresorcin;
N-(4-Amino-3-methylphenyl)-N-[3-(1H-imidazol-1-yl)propyl]amin / 5-Amino-2-methylphenol;
N-(4-Amino-3-methylphenyl)-N-[3-(1H-imidazol-1-yl)propyl]amin / 3-Aminophenol;
N-(4-Amino-3-methylphenyl)-N-[3-(1H-imidazol-1-yl)propyl]amin / 2-(2,4-Diaminophenoxy)ethanol;
N-(4-Amino-3-methylphenyl)-N-[3-(1H-imidazol-1-yl)propyl]amin / 1,3-Bis(2,4-diaminophenoxy)propan;
N-(4-Amino-3-methylphenyl)-N-[3-(1H-imidazol-1-yl)propyl]amin / 1-Methoxy-2-amino-4-(2-hydroxy-ethylamino)benzol;
N-(4-Amino-3-methylphenyl)-N-[3-(1H-imidazol-1-yl)propyl]amin / 2-Amino-3-hydroxypyridin;
N-(4-Amino-3-methylphenyl)-N-[3-(1H-imidazol-1-yl)propyl]amin / 1-Naphthol;
4,5-Diamino-1-(2-hydroxyethyl)pyrazol / Resorcin;
4,5-Diamino-1-(2-hydroxyethyl)pyrazol / 2-Methylresorcin;
4,5-Diamino-1-(2-hydroxyethyl)pyrazol / 5-Amino-2-methylphenol;
4,5-Diamino-1-(2-hydroxyethyl)pyrazol / 3-Aminophenol;
4,5-Diamino-1-(2-hydroxyethyl)pyrazol / 2-(2,4-Diaminophenoxy)ethanol;
4,5-Diamino-1-(2-hydroxyethyl)pyrazol / 1,3-Bis(2,4-diaminophenoxy)propan;
4,5-Diamino-1-(2-hydroxyethyl)pyrazol / 1-Methoxy-2-amino-4-(2-hydroxyethylamino)benzol;
4,5-Diamino-1-(2-hydroxyethyl)pyrazol / 2-Amino-3-hydroxypyridin;
4,5-Diamino-1-(2-hydroxyethyl)pyrazol / 1-Naphthol.

Um eine ausgewogene und subtile Nuancenausbildung zu erzielen, ist es erfindungsgemäß vorteilhaft, wenn weitere farbgebende Komponenten in dem Mittel enthalten sind, das in Schritt b) dieser Variante des erfindungsgemäßen Verfahrens eingesetzt wird.

In einer weiteren Ausführungsform können die in Schritt b) dieser Variante des erfindungsgemäßen Verfahrens eingesetzten Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die Oxidationsmittel, die die Mittel, welche in Schritt b) dieser Variante des erfindungsgemäßen Verfahrens eingesetzt werden, enthalten, sind analog denen, die bei der Blondier-Variante des erfindungsgemäßen Verfahrens beschrieben wurden.

Ein weiterer Gegenstand der vorliegenden Erfindung ist ein Verfahren zum Aufhellen von Keratinfasern, insbesondere menschlichen Haaren, dadurch gekennzeichnet, daß
- ein Vorbehandlungsmittel auf die keratinischen Fasern aufgetragen wird, das bezogen auf sein Gewicht
   a) 0,00001 bis 20 Gew.-% mindestens eines 4-morpholinomethyl-substituierten Silikons enthält, das jeweils mindestens eine der Struktureinheiten der Formeln (I), (II) und (III) aufweist 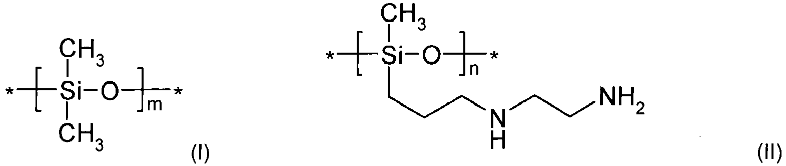 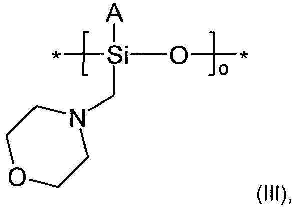 worin
      - *: für eine Bindung zu einer der Struktureinheiten (I), (II) oder (III) oder für eine Endgruppe B (Si-gebunden) oder D (O-gebunden) steht,
      - B: für eine Gruppe -OH, -O-Si(CH₃)₃, -O-Si(CH₃)₂OH, -O-Si(CH₃)₂OCH₃ steht,
      - D: für eine Gruppe -H; -Si(CH₃)₃, -Si(CH₃)₂OH, -Si(CH₃)₂OCH₃ steht,
      - A: für eine über ein -O- gebundene Struktureinheit (I) oder einen über ein -O- gebundenen oligomeren oder polymeren Rest enthaltend Struktureinheiten der Formel (I) oder für -OH steht,
      - n, m und o: für ganze Zahlen zwischen 1 und 1000 stehen.
   b) mindestens 50 Gew.-% Wasser enthält
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-60 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M2, M3 oder M4 mindestens ein Oxidationsmittel mindestens eine Peroxoverbindung, vorzugsweise Wasserstoffperoxid, enthält.

Ein weiterer Gegenstand der vorliegenden Erfindung ist ein Verfahren zum Aufhellen von Keratinfasern, insbesondere menschlichen Haaren, dadurch gekennzeichnet, daß
- ein Vorbehandlungsmittel auf die keratinischen Fasern aufgetragen wird, das bezogen auf sein Gewicht
   a) 0,00001 bis 20 Gew.-% mindestens eines 4-morpholinomethyl-substituierten Silikons enthält, das jeweils mindestens eine der Struktureinheiten der Formeln (I), (II) und (III) aufweist 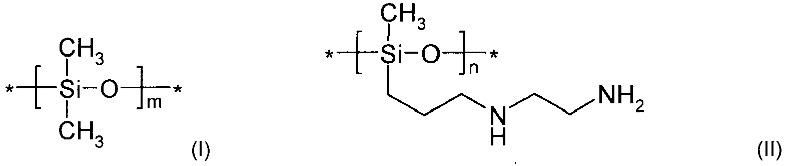 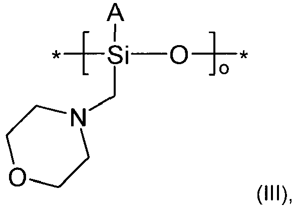 worin
      - *: für eine Bindung zu einer der Struktureinheiten (I), (II) oder (III) oder für eine Endgruppe B (Si-gebunden) oder D (O-gebunden) steht,
      - B: für eine Gruppe -OH, -O-Si(CH₃)₃, -O-Si(CH₃)₂OH, -O-Si(CH₃)₂OCH₃ steht,
      - D: für eine Gruppe -H; -Si(CH₃)₃, -Si(CH₃)₂OH, -Si(CH₃)₂OCH₃ steht,
      - A: für eine über ein -O- gebundene Struktureinheit (I) oder einen über ein -O- gebundenen oligomeren oder polymeren Rest enthaltend Struktureinheiten der Formel (I) oder für-OH steht,
      - n, m und o: für ganze Zahlen zwischen 1 und 1000 stehen.
   b) mindestens 50 Gew.-% Wasser enthält
- ein Mittel M2 auf der Faser zur Anwendung kommt, das gewünschtenfalls vor der Anwendung aus einem Aufhellmittel M2a und einem Oxidationsmittel M2b gemischt wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird.

Die im erfindungsgemäßen Verfahren eingesetzten Mittel können demnach als Einkomponentenmittel (Mittel M2), oder als Zweikomponenten Mittel (M2a + M2b) formuliert und entsprechend angewendet werden.

Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Aufhellungsverfahren, bei dem das Aufhellmittel M2a und das Oxidationsmittel M2b zunächst getrennt vorliegen, ist dabei bevorzugt.

Für weitere bevorzugte Ausführung gilt mutatis mutandis das zu den erfindungsgemäßen Mitteln Gesagte.

## Patentansprüche

1. Verfahren zur oxidativen Haarbehandlung, **dadurch gekennzeichnet, daß**
a) ein Vorbehandlungsmittel auf die keratinischen Fasern aufgetragen wird, das bezogen auf sein Gewicht
a) 0,00001 bis 20 Gew.-% mindestens eines 4-morpholinomethyl-substituierten Silikons enthält, das jeweils mindestens eine der Struktureinheiten der Formeln (I), (II) und (III) aufweist 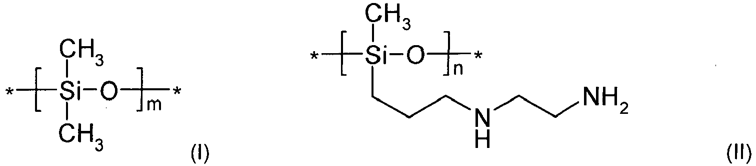 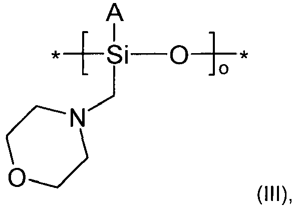 worin
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃, -O-Si(CH₃)₂OH, -O-Si(CH₃)₂OCH₃ steht,
D für eine Gruppe -H; -Si(CH₃)₃, -Si(CH₃)₂OH, -Si(CH₃)₂OCH₃ steht,
A für eine über ein -O- gebundene Struktureinheit (I) oder einen über ein -O- gebundenen oligomeren oder polymeren Rest enthaltend Struktureinheiten der Formel (I) oder für-OH steht,
n, m und o für ganze Zahlen zwischen 1 und 1000 stehen.
b) mindestens 50 Gew.-% Wasser enthält
b) die keratinischen Fasern innerhalb eines Zeitraumes von einer Sekunde bis 24 Stunden nach Schritt a) einer oxidativen Haarbehandlung unterworfen werden.

2. Verfahren nach Anspruch 1, **dadurch gekennzeichnet, daß** das Vorbehandlungsmittel, bezogen auf das Gewicht des Mittels, 70 bis 99,9 Gew.%, vorzugsweise 80 bis 99,5 Gew.%, besonders bevorzugt 85 bis 99,25 Gew.% und insbesondere 90 bis 98 Gew.% Wasser enthält.

3. Verfahren nach einem der Ansprüche 1 oder 2, **dadurch gekennzeichnet, daß** das Vorbehandlungsmittel mindestens ein 4-morpholinomethyl-substituiertes Silikon der Formel (V) enthält 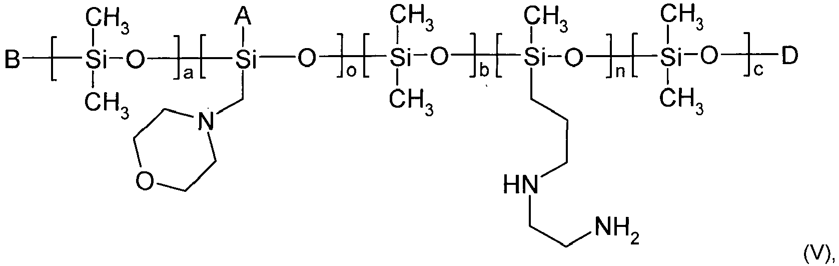 in der
A für eine über ein -O- gebundene Struktureinheit (I) oder einen über ein -O-gebundenen oligomeren oder polymeren Rest enthaltend Struktureinheiten der Formel (I) oder für-OH steht,
B für eine Gruppe -OH, -O-Si(CH₃)₃, -O-Si(CH₃)₂OH, -O-Si(CH₃)₂OCH₃ steht,
D für eine Gruppe -H; -Si(CH₃)₃, -Si(CH₃)₂OH, -Si(CH₃)₂OCH₃ steht,
a, b und c für ganze Zahlen zwischen 0 und 1000 stehen, mit der Maßgabe a + b + c > 0
n und o für ganze Zahlen zwischen 1 und 1000 stehen.

4. Verfahren nach einem der Ansprüche 1 bis 3, **dadurch gekennzeichnet, daß** das Vorbehandlungsmittel mindestens ein 4-morpholinomethyl-substituiertes Silikon enthält, bei dem
- m > (n + o) bzw. (a+b+c) > (n + o), vorzugsweise
- m > 10(n + o) bzw. (a+b+c) > 10(n + o), besonders bevorzugt
- m > 50(n + o) bzw. (a+b+c) > 50(n + o), weiter bevorzugt
- m > 75(n + o) bzw. (a+b+c) > 75(n + o) und insbesondere
- m > 100(n + o) bzw. (a+b+c) > 100(n + o) gilt.

5. Verfahren nach einem der Ansprüche 1 bis 4, **dadurch gekennzeichnet, daß** Schritt b) das Aufbringen eines Mittels zum Blondieren menschlicher Haare umfaßt, welches mindestens eine Peroxoverbindung enthält, die ausgewählt ist aus Wasserstoffperoxid und dessen Anlagerungsverbindungen an feste Träger, Ammonium- und Alkalimetall-persulfaten und -peroxidisulfaten.

6. Verfahren nach einem der Ansprüche 1 bis 5, **dadurch gekennzeichnet, daß** Schritt b) das Aufbringen eines Haarfärbemittels umfaßt, welches mindestens ein Oxidationsfarbstoffvorprodukt und mindestens ein Oxidationsmittel enthält.

7. Verfahren zum Aufhellen von Keratinfasern, insbesondere menschlichen Haaren, **dadurch gekennzeichnet, daß**
- ein Vorbehandlungsmittel auf die keratinischen Fasern aufgetragen wird, das bezogen auf sein Gewicht
a) 0,00001 bis 20 Gew.-% mindestens eines 4-morpholinomethyl-substituierten Silikons enthält, das jeweils mindestens eine der Struktureinheiten der Formeln (I), (II) und (III) aufweist 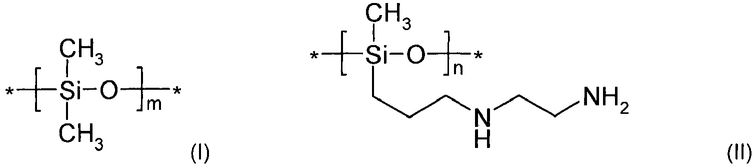 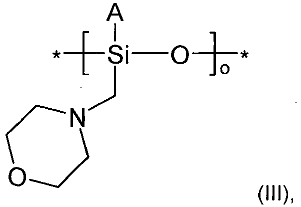 worin
* für eine Bindung zu einer der Struktureinheiten (I), (II) oder (III) oder für eine Endgruppe B (Si-gebunden) oder D (O-gebunden) steht,
B für eine Gruppe -OH, -O-Si(CH₃)₃, -O-Si(CH₃)₂OH, -O-Si(CH₃)₂OCH₃ steht,
D für eine Gruppe -H; -Si(CH₃)₃, -Si(CH₃)₂OH, -Si(CH₃)₂OCH₃ steht,
A für eine über ein -O- gebundene Struktureinheit (I) oder einen über ein -O- gebundenen oligomeren oder polymeren Rest enthaltend Struktureinheiten der Formel (I) oder für-OH steht,
n, m und o für ganze Zahlen zwischen 1 und 1000 stehen.
b) mindestens 50 Gew.-% Wasser enthalt, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-60 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird, wobei mindestens eines der Mittel M2, M3 oder M4 mindestens ein Oxidationsmittel mindestens eine Peroxoverbindung, vorzugsweise Wasserstoffperoxid, enthält.

## Claims

1. A method for oxidative hair treatment, **characterized in that**
a) a pre-treatment agent is applied to the keratin fibers and contains, based on its weight,
a) 0.00001 to 20 wt.% of at least one 4-morpholinomethyl-substituted silicone that comprises in each case at least one of the structural units of formulae (I), (II) and (III), 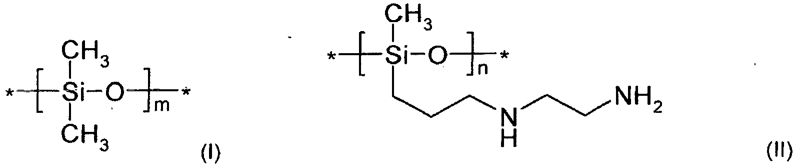 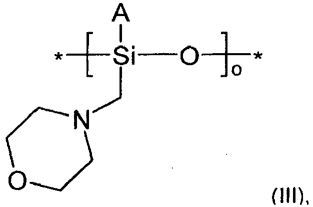 where
* represents a bond to one of the structural units (I), (II) or (III) or represents a terminal group B (Si-bound) or D (O-bound),
B represents a group -OH, -O-Si(CH₃)₃, -O-Si(CH₃)₂OH, -O-Si(CH₃)₂OCH₃,
D represents a group -H; -Si(CH₃)₃, -Si(CH₃)₂OH, -Si(CH₃)₂OCH₃,
A represents a structural unit (I) bound via an -O- or represents an oligomeric or polymeric group bound via an -O- and containing structural units of formula (I) or represents -OH,
n, m and o represent integers between 1 and 1000.
b) at least 50 wt.% water
b) after step a), the keratin fibers are subject to oxidative hair treatment within a period of one second to 24 hours.

2. The method according to claim 1, **characterized in that** the pre-treatment agent contains, based on the weight of the agent, 70 to 99.9 wt.%, preferably 80 to 99.5 wt.%, particularly preferably 85 to 99.25 wt.%, and in particular 90 to 98 wt.%, water.

3. The method according to claim 1 or 2, **characterized in that** the pre-treatment agent contains at least one 4-morpholinomethyl-substituted silicone of formula (V) 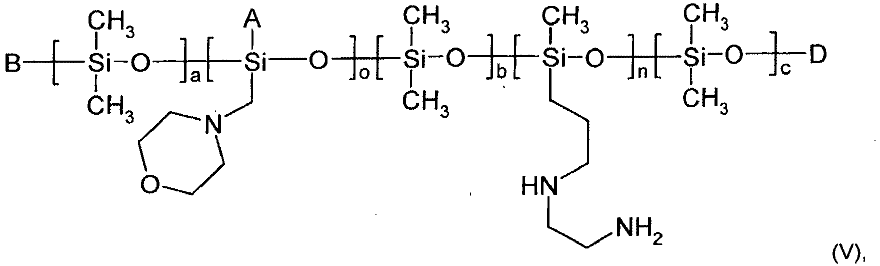 where
A represents a structural unit (I) bound via an -O- or represents an oligomeric or polymeric group bound via an -O- and containing structural units of formula (I) or represents -OH,
B represents a group -OH, -O-Si(CH₃)₃, -O-Si(CH₃)₂OH, -O-Si(CH₃)₂OCH₃,
D represents a group -H; -Si(CH₃)₃, -Si(CH₃)₂OH, -Si(CH₃)₂OCH₃,
a, b and c represent integers between 0 and 1000, with the proviso that a + b + c > 0
n and o represent integers between 1 and 1000.

4. The method according to one of claims 1 to 3, **characterized in that** the pre-treatment agent contains at least one 4-morpholinomethyl-substituted silicone, where
- m > (n + o) or (a+b+c) > (n+o), preferably
- m > 10(n + o) or (a+b+c) > 10(n + o), particularly preferably
- m > 50(n + o) or (a+b+c) > 50(n + o), more preferably
- m > 75(n + o) or (a+b+c) > 75(n + o), and in particular
- m > 100(n + o) or (a+b+c) > 100(n + o), hold.

5. The method according to one of claims 1 to 4, **characterized in that** step b) comprises applying an agent for bleaching human hair that contains at least one peroxo compound selected from hydrogen peroxide and addition compounds thereof to solid carriers, ammonium persulfate, alkali metal persulfates, ammonium peroxydisulfate, and alkali metal peroxydisulfates.

6. The method according to one of claims 1 to 5, **characterized in that** step b) comprises applying a hair dye that contains at least one oxidation dye precursor and at least one oxidizing agent.

7. A method for lightening keratin fibers, in particular human hair, **characterized in that**
- a pre-treatment agent is applied to the keratin fibers, which contains, based on its weight,
a) 0.00001 to 20 wt.% of at least one 4-morpholinomethyl-substituted silicone that comprises in each case at least one of the structural units of formulae (I), (II) and (III) 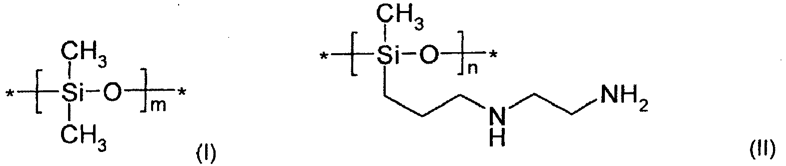 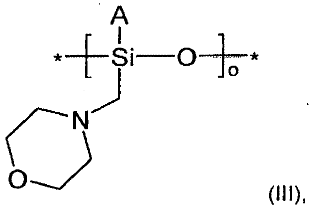 where
* represents a bond to one of the structural units (I), (II) or (III) or represents a terminal group B (Si-bound) or D (O-bound),
B represents a group -OH, -O-Si(CH₃)₃, -O-Si(CH₃)₂OH, -O-Si(CH₃)₂OCH₃,
D represents a group -H; -Si(CH₃)₃, -Si(CH₃)₂OH, -Si(CH₃)₂OCH₃,
A represents a structural unit (I) bound via an -O- or represents an oligomeric or polymeric group bound via an -O- and containing structural units of formula (I) or represents -OH,
n, m and o represent integers between 1 and 1,000.
b) at least 50 wt.% water, then
- an agent M2 is used on the fiber, an additional agent M3 being added, if desired, to the agent M2 before said agent is used,
- said agent M2 is rinsed off the fiber after 5-60 minutes,
- and a post-treatment agent M4 is optionally applied to the fibers after treatment and is rinsed off again after a contact time of a few minutes, at least one of the agents M2, M3 or M4 containing at least one oxidizing agent, namely at least one peroxo compound, preferably hydrogen peroxide.

## Revendications

1. Procédé de traitement des cheveux par oxydation, **caractérisé en ce que** :
a) on applique un produit de traitement préalable sur les fibres de kératine, qui contient, rapporté à son poids :
a) 0,00001 à 20 % en poids d'au moins un silicone substitué 4-morpholinométhyle, présentant respectivement au moins une des unités structurelles de formule (I), (II) ou (III) : 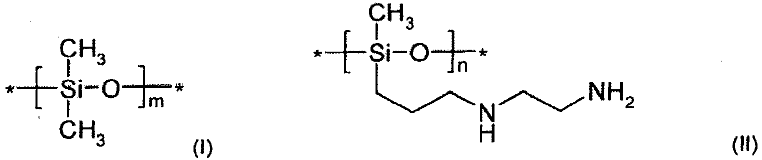 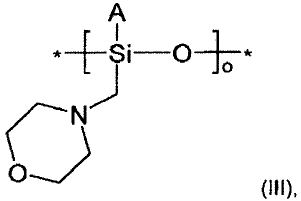 où
* représente une liaison à une des unités structurelles (I), (II) ou (III) ou un groupe terminal B (lié à un atome Si) ou D (lié à un atome O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
A représente une unité structurelle (I) liée par un atome O ou un résidu oligomère ou polymère lié par un atome O et contenant des unités structurelles de formule (I), ou -OH,
n, m et o représentent des entiers entre 1 et 1 000,
b) au moins 50 % en poids d'eau,
b) les fibres de kératine sont soumises à un traitement capillaire oxydant sur une période entre 1 s et 24 h après l'étape a).

2. Procédé selon la revendication 1, caractérisé en le que le traitement préalable contient, rapporté au poids du produit, 70 à 99,9 % en poids, de préférence 80 à 99,5 % en poids, particulièrement préférentiellement 85 à 99,25 % et en particulier 90 à 98 % en poids d'eau.

3. Procédé selon une des revendications 1 ou 2, **caractérisé en ce que** le traitement préalable contient au moins un silicone substitué 4-morpholinométhyle de formule (V) : 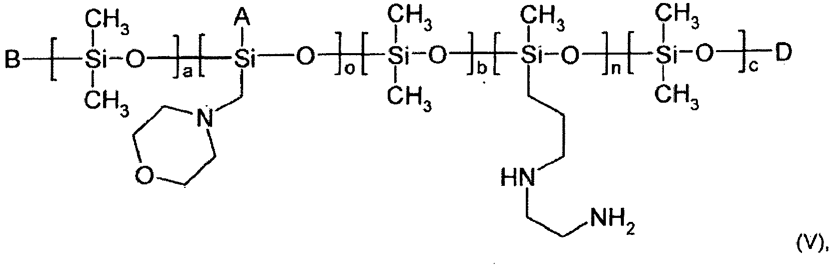 où
A représente une unité structurelle (I) liée par un atome O ou un résidu oligomère ou polymère lié par un atome O et contenant des unités structurelles de formule (I), ou -OH,
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
a, b et c représentent des entiers entre 0 et 1 000, à condition que a + b + c > 0,
n et o représentent des entiers entre 1 et 1 000.

4. Procédé selon l'une des revendications 1 à 3, **caractérisé en ce que** le produit de traitement préalable contient au moins une silicone substitué 4-morpholinométhyle, où :
- m > (n + o) ou resp. a + b + c > n + o, de préférence
- m > 10(n + o) ou resp. a + b + c > 10(n + o), particulièrement préférentiellement
- m > 50(n + o) ou resp. a + b + c > 50(n + o), plus préférentiellement
- m > 75(n + o) ou resp. a + b + c > 75(n + o), et en particulier
- m > 100(n + o) ou resp. a + b + c > 100(n + o).

5. Procédé selon l'une des revendications 1 à 4, **caractérisé en ce que** l'étape b) comprend l'application d'un produit pour blondir les cheveux humains, qui contient un peroxyde choisi parmi le peroxyde d'hydrogène et ses compositions d'addition sur des supports solides, des persulfates et peroxodisulfates d'ammonium et de métaux alcalins.

6. Procédé selon l'une des revendications 1 à 5, **caractérisé en ce que** l'étape b) comprend l'application d'un produit pour colorer les cheveux, qui contient un précurseur de coloration par oxydation et au moins un oxydant.

7. Procédé d'éclaircissement de fibres de kératine, en particulier de cheveux humains, **caractérisé en ce que** :
- on applique sur les fibres de kératine un produit de traitement préalable qui contient, rapporté à son poids :
a) 0,00001 à 20 % en poids d'au moins un silicone substitué 4-morpholinométhyle, présentant respectivement au moins une des unités structurelles de formule (I), (II) ou (III) : 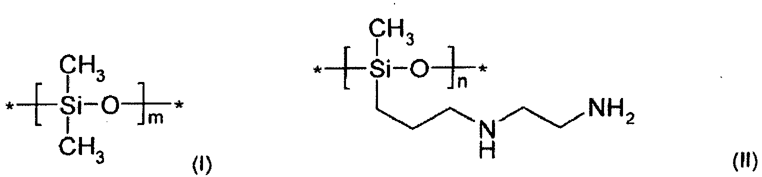 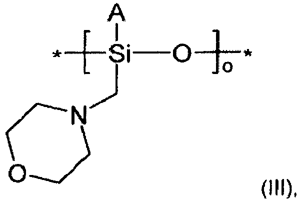 où
* représente une liaison à une des unités structurelles (I), (II) ou (III) ou un groupe terminal B (lié à un atome Si) ou D (lié à un atome O),
B représente un groupe -OH, -O-Si(CH₃)₃, -O-Si(CH₃)₂OH, -O-Si(CH₃)₂OCH₃,
D représente un groupe -H, -Si(CH₃)₃, -Si(CH₃)₂OH, -Si(CH₃)₂OCH₃,
A représente une unité structurelle (I) liée par un atome O ou un résidu oligomère ou polymère lié par un atome O et contenant des unités structurelles de formule (I), ou -OH,
n, m et o représentent des entiers entre 1 et 1 000,
b) au moins 50 % en poids d'eau,
- on utilise sur les fibres un produit M2, on ajoute si on le désire un autre produit M3 au produit M2 avant utilisation,
- on rince ledit produit M2 des fibres après une durée de 5 à 60 minutes,
- et après le traitement, éventuellement, on applique un produit après traitement M4 sur les fibres, et on le rince après une durée d'action de quelques minutes,
où au moins un des produits M2, M3 ou M4 contient au moins un oxydant, au moins un peroxyde, de préférence le peroxyde d'hydrogène.
